# FASCICULE DE BREVET EUROPEEN

(11) **EP 3 209 318 B1**
(45) Date de publication et mention de la délivrance du brevet: **29.04.2020**
(21) Numéro de dépôt: 15786908.2
(22) Date de dépôt: 23.10.2015
(51) Int. Cl.: A61K 38/16, A61K 9/00, A61K 49/00, C07K 14/195, A61P 1/00

(54) **COMPOSITIONS POUR ADMINISTRATION PAR VOIE ORALE COMPRENANT UN VARIANT D'UNE PROTÉINE À PLI-OB**
ZUSAMMENSETZUNGEN ZUR ORALEN VERABREICHUNG MIT EINER OB-FOLD-PROTEINVARIANTE
ORAL ADMINISTRATION COMPOSITIONS COMPRISING AN OB-FOLD PROTEIN VARIANT

(30) Priorité: 24.10.2014 FR 1460226
(43) Date de publication de la demande: 30.08.2017
(73) Titulaire: AFFILOGIC, 44200 Nantes (FR)
(72) Inventeur: KITTEN, Olivier, F-44000 Nantes (FR)
(74) Mandataire: Flesselles, Bruno F.G.
(86) Numéro de dépôt international: PCT/EP2015/074644
(87) Numéro de publication internationale: WO 2016/062874

(56) Documents cités:
- WO-A1-2012/150314
- WO-A1-2014/173899
- WO-A2-2008/068637
- NIMISH GERA ET AL: "Highly Stable Binding Proteins Derived from the Hyperthermophilic Sso7d Scaffold", JOURNAL OF MOLECULAR BIOLOGY, ACADEMIC PRESS, UNITED KINGDOM, vol. 409, no. 4, 8 avril 2011 (2011-04-08) , pages 601-616, XP028221838, ISSN: 0022-2836, DOI: 10.1016/J.JMB.2011.04.020 [extrait le 2011-04-16]

## Description

L'invention se rapporte au domaine de la préparation de compositions orales pour un usage thérapeutique, contenant des principes actifs se liant à des cibles d'intérêt.

Il existe un certain nombre de maladies du système gastro-intestinal. Afin de traiter ces maladies, il est souhaitable de pouvoir disposer de compositions agissant directement au lieu de la maladie.

Pour ce faire, il est possible de développer des compositions par voie rectale, mais les compositions par voie orale sont préférées des patients et permettent un meilleur respect du traitement. Par ailleurs, et même pour traiter les maladies systémiques (non localisées dans le système gastro-intestinal), l'administration par voie orale est préférée, dans la mesure du possible aux autres modes d'administration (et notamment les injections), qui peuvent demander l'implication de membres du personnel soignant.

Quelle que soit la maladie, il existe généralement une cible d'intérêt thérapeutique sur laquelle les médicaments sont actifs. Ce peut être un récepteur cellulaire, ou une protéine de surface d'un microorganisme. D'une façon générale, il apparaît souhaitable de disposer de compositions permettant l'application de principes actifs agissant sur les cibles d'intérêt thérapeutiques directement sur le lieu d'action. Toutefois, certaines protéines, comme les anticorps, sont dégradées lors du passage dans l'estomac.

La demande WO 2007/139397 décrit l'utilisation de banques de protéines à pli-OB dans lesquelles on modifie le domaine OB par l'introduction de mutations dans ce domaine de liaison de la protéine à son ligand naturel.

La demande WO 2008/068637 décrit l'utilisation d'une banque basée sur la protéine Sac7d pour obtenir des ligands présentant une affinité pour des cibles d'intérêt. La méthode décrite dans WO 2008/068637 comprend la génération de banques combinatoires contenant une pluralité de molécules d'ADN ayant toutes la même séquence, sauf la présence de certaines mutations aléatoires menant à la production de variants d'une protéine sauvage, présentant des mutations à certains acides aminés du site de liaison de cette protéine à pli-OB sauvage. En particulier, dans le cadre de WO 2008/068637, la protéine à pli-OB sauvage est une protéine Sac7d, dans laquelle on introduit des mutations pour générer une variabilité, notamment aux acides aminés choisis parmi K7, Y8, K9, K21, K22, W24, V26, M29, S31, T33, T40, R42, A44, S46, ou sur d'autres acides aminés, comme V26, G27, K28, M29, S31, R42, A44, S46, E47 et K48. Ces acides aminés sont basés sur la séquence de Sac7d, telle que représentée par SEQ ID N° 1.

La demande de brevet WO 2012/150314 présente la portabilité de mutations d'une protéine de la famille Sac7d vers une autre protéine de la même famille. Cette portabilité revient à créer un mutant d'une autre protéine de la famille Sac7d à partir d'un mutant d'une protéine de ladite famille, que l'on a pu obtenir notamment en mettant en œuvre le procédé de WO 2008/068637.

La famille Sac7d est définie comme se rapportant à La famille Sac7d et correspond à une famille de protéines de 7 kDa liant l'ADN isolées de bactéries extrémophiles. Ces protéines et cette famille sont notamment décrites dans WO 2008/068637. Ainsi, dans le cadre de la présente invention, une protéine appartient à la famille Sac7d lorsqu'elle présente une séquence correspondant à la séquence SEQ ID N° 8. Cette famille comprend notamment les protéines Sac7d ou Sac7e issues de *Sulfolobus acidocaldarius,* Sso7d issue de *Sulfolobus solfataricus,* DBP 7 issue de *Sulfolobus tokodaii,* Ssh7b issue de *Sulfolobus shibatae,* Ssh7a issue de *Sulfolobus shibatae,* et p7ss issue de *Sulfolobus solfataricus.*

Les protéines à pli-OB sont connues dans l'art. Elles sont notamment décrites dans les documents cités plus haut, ainsi que dans Arcus (Curr Opin Struct Biol. 2002 Dec;12(6):794-801). Le pli-OB se présente sous la forme d'un cylindre présentant cinq feuillets beta (β). La plupart des protéines à pli-OB utilisent la même interface de liaison de leur ligand naturel, qui peut être un oligosaccharide, un oligonucléotide, une protéine, un ion métallique ou un substrat catalytique. Cette interface de liaison comprend principalement les résidus situés dans les feuillets beta. Certains résidus situés dans les boucles peuvent aussi être impliqués dans la liaison d'une protéine à pli-OB avec son ligand naturel. Ainsi, les demandes WO 2007/139397 et WO 2008/068637 et le document Arcus (2002, *op. cit*.) décrivent les domaines de liaison des protéines à pli-OB avec leur ligand naturel. Ainsi, le document WO 2008/068637 décrit précisément la façon d'identifier le domaine de liaison d'une protéine à pli-OB.

En superposant plusieurs séquences et structures 3D de protéines présentant des domaines à pli-OB en utilisant les sites Web WU-Blast2 (http://www.ebi.ac.uk/blast2/index.html) (Lopez et al., 2003, Nucleic Acids Res 31, 3795-3798), T-COFFEE (http://www.ch.embnet.org/software/TCoffee.html) (Notredame et al., 2000, J Mol Biol 302, 205-217) et DALI lite (http://www.ebi.ac.uk/DaliLite/) (Holm et Park, 2000, Bioinformatics 16, 566-567) on peut identifier les positions des domaines de liaison et notamment les acides aminés qui peuvent être modifiés. En prenant comme référence la séquence de Sac7d (SEQ ID N °1), il s'agit des résidus V2, K3, K5, K7, Y8, K9, G10, E11, K13, E14, T17, K21, K22, W24, V26 , G27, K28, M29, S31, T33, Y34, D36, N37, G38, K39, T40, R42, A44, S46, E47, K48, D49, A50 et P51.
Toujours avec cette séquence Sac7d en tant que référence, les résidus qui peuvent être supprimés sont: A59, R60, A61, E64 et/ou K66.

L'identification des domaines de liaison d'autres protéines à pli-OB peut être effectuée, ainsi que décrit dans WO 2008/068637. Cette demande décrit que l'on peut effectuer une superposition de structures 3D de protéines ou domaines pli-OB (10 domaines ont été utilisés dans cette demande, y compris Sac7d), en utilisant le site Web DALI (http://www.ebi.ac.uk/dali/lnteractive.html) (Holm et Sander, 1998, Nucleic Acids Res 26, 316-319). Ainsi, il est aisé d'identifier, pour toute protéine (ou tout domaine) à pli-OB, les acides aminés impliqués dans le site de liaison et correspondant aux acides aminés de Sac7d mentionnés ci-dessus.

L'enseignement de WO 2008/068637 indique également que l'on peut éventuellement insérer des acides aminés dans les boucles des protéines à pli-OB, notamment les protéines de la famille Sac7d, en particulier des insertions de 1 à 15 résidus d'acides aminés peuvent être effectuées dans la boucle 3 (telle que définie dans les figures 1b et 2 de WO 2008/068637), par exemple dans la région des résidus 25 à 30 de Sac7d, de préférence entre les résidus 27 et 28, des insertions de 1 à 15 résidus d'acides aminés peuvent être effectuées dans la boucle 4 (telle que définie dans les figures 1b et 2 de WO 2008/068637), par exemple dans la région des résidus 35-40 de Sac7d, de préférence entre les résidus 37 et 38, et des insertions de 1 à 20 résidus peuvent être effectuée dans la boucle 1 (telle que définie dans les figures 1b et 2 de WO 2008/068637), par exemple dans la région des résidus 7 à 12 de Sac7d, de préférence entre les résidus 9 et 10.

Par extension, dans le cadre de la présente demande, le terme « protéine à pli-OB » comprend les protéines à pli-OB naturelles, mais également les domaines présentant un pli-OB que l'on peut isoler de protéines plus complexes. Ces domaines à pli-OB sont notamment décrits plus en détail dans les demandes WO 2007/139397 et WO 2008/068637. Ce terme comprend également les polypeptides que l'on peut obtenir en fusionnant, par génie génétique, en N ou C-terminal, une protéine à pli-OB ou un domaine présentant un pli-OB à une protéine ou un domaine d'intérêt, par exemple une étiquette permettant une meilleure purification.

L'intérêt de la méthode décrite dans WO 2008/068637 est qu'elle permet d'obtenir des variants de protéines à pli-OB par criblage de banques combinatoires contenant ou exprimant une pluralité de variants dans lesquels un certain nombre d'acides aminés ont été « randomisés », c'est-à-dire remplacés par un acide aminé aléatoire. Le criblage de ces banques permet d'identifier des variants de ces protéines qui se lient de façon spécifique, généralement avec une forte affinité (la demande WO 2008/068637 décrit en effet des affinités de l'ordre du nanomolaire), avec une cible d'intérêt, différente du ligand naturel de la protéine sauvage à partir de laquelle la banque combinatoire a été générée.

Les inventeurs ont montré que les protéines à pli-OB sont suffisamment résistantes à la dégradation gastrique, et peuvent rester dans l'intestin ou traverser la barrière de l'intestin et être donc utilisées dans des compositions utilisables par voie orale pour le traitement de diverses maladies, notamment localisées au niveau de l'intestin, ou dans d'autres organes du corps, après passage dans la circulation systémique. On utilise de préférence un variant d'une protéine à pli-OB, qui se lie à une cible d'intérêt, ladite cible d'intérêt étant impliquée dans une pathologie, notamment du système gastro-intestinal ou une pathologie systémique (c'est-à-dire une pathologie localisée dans un organe autre que le système gastro-intestinal).

La présente invention se rapporte ainsi à une composition pour utilisation thérapeutique ou diagnostic par administration par voie orale comprenant une protéine à pli-OB ou un variant d'une protéine sauvage à pli-OB, ledit variant présentant entre 5 et 32 résidus mutés dans l'interface de liaison de ladite protéine sauvage à pli-OB à son ligand naturel.

Dans un mode de réalisation particulier, ledit variant présente une activité thérapeutique. En particulier, ledit variant se lie de façon spécifique à une cible d'intérêt différente de la cible dudit ligand naturel. Ce variant peut alors avoir été identifié par la mise en œuvre d'une méthode telle que décrite dans WO 2008/068637 ou WO 2007/139397. De fait, la mise en œuvre des méthodes décrites dans ces deux demandes de brevet permet généralement d'identifier des variants de toute protéine à pli-OB se liant à toute cible d'intérêt. Cette composition contient également généralement un excipient utilisable pour une telle administration par voie orale, et pharmaceutiquement acceptable

Ainsi que vu plus haut, ladite protéine sauvage à pli-OB englobe aussi les domaines à pli-OB. D'une façon préférée, le variant mis en œuvre dans le cadre de la présente invention contient au maximum 300 acides aminés, de préférence au maximum 200 acides aminés, de préférence au maximum 175 acides aminés, de façon plus préférée au maximum 150 acides aminés, de façon plus préférée au maximum 100 acides aminés. Dans un mode de réalisation particulier, il contient au maximum 80 ou au maximum 70 acides aminés.

Le nombre de résidus mutés dans ledit variant (par rapport à la protéine sauvage) est compris entre 5 et 32. Dans d'autres modes de réalisation, ces variants présentent de préférence au moins 5, de façon plus préférée au moins 8, de façon encore plus préférée au moins 10, mais généralement moins de 32, de façon plus préférée moins de 24, de façon encore plus préférée moins de 20 ou moins de 15 acides aminés substitués par rapport à la protéine (ou au domaine) à pli-OB sauvage. On préfère lorsque 8, 9, 10, 11, 12, 13 ou 14 acides aminés sont mutés par rapport à la protéine sauvage. Ces acides aminés mutés sont localisés dans le site de liaison de la protéine à pli-OB avec son ligand naturel. Ils sont généralement répartis sur l'ensemble de ce domaine de liaison. Étant donné la structure de ce domaine de liaison, certains résidus mutés se trouvent dans un (généralement plusieurs, notamment deux ou trois) feuillet beta.

Dans un mode de réalisation particulier, ces variants peuvent également comprendre des insertions d'acides aminés dans des boucles reliant les feuillets beta du pli-OB. Ainsi, on peut introduire entre 1 et 15 acides aminés dans la boucle 1 et/ou dans la boucle 4 et/ou dans la boucle 3 (les boucles étant numérotées de la même façon que dans WO 2008/068637.

Dans un mode de réalisation particulier, ladite protéine sauvage à pli-OB est choisie parmi Sac7d or Sac7e issues de *Sulfolobus acidocaldarius,* Sso7d issue de *Sulfolobus solfataricus,* DBP 7 issue de *Sulfolobus tokodaii,* Ssh7b issue de *Sulfolobus shibatae,* Ssh7a issue de *Sulfolobus shibatae,* et p7ss issue de *Sulfolobus solfataricus.*

Il s'agit donc de la protéine à laquelle est comparée le variant mis en œuvre dans la composition pour administration par voie orale de la présente invention.

Les différentes séquences des protéines Sac7d, Sso7d, Sac7e, Ssh7b, SSh7a, DBP7 et Sis7 sont représentées par SEQ ID N° 1 à SEQ ID N° 7 respectivement.

Un variant d'une protéine de cette famille Sac7d est appelée une nanofitine. La mise en œuvre de l'invention se fait ainsi préférentiellement sur des variants des protéines représentées par SEQ ID N° 1 à SEQ ID N° 7, en particulier sur des variants de Sac7d.

Dans le cas d'une composition liquide, la concentration du variant dans la composition selon la présente invention est généralement supérieure à 10 ng/ml et inférieure à 600 mg/ml. Elle est donc généralement inférieure à 500 mg/ml, de préférence inférieure 250 mg/ml, de préférence inférieure à 100 mg/ml, ou à 50 mg / ml, voire inférieure à 10 mg/ml. Elle est généralement supérieure à 10 ng / ml, de préférence supérieure à 50 ng / ml, de préférence supérieure à 100 ng / ml.

La concentration est adaptée à la dose que l'on souhaite administrer au patient pour une prise comprise entre 5 et 20 ml.

Pour une composition solide, la dose est adaptée en fonction du nombre de comprimés (ou tablettes ou autres) que prendra le patient pour obtenir la dose efficace d'un point de vue thérapeutique.

D'une façon générale, on administre au patient une quantité très variable (qui va dépendre notamment de la nature de la maladie, de l'antigène visé, éventuellement de l'affinité du variant de la protéine à pli-OB contre cette antigène, du poids du patient, de l'utilisation en combinaison ou non avec d'autres médicaments pour la pathologie). La gamme ira donc généralement de 10 mg/kg jusqu'à 200 mg/kg, voire 400 mg/kg du variant de la protéine à pli-OB en une ou plusieurs prises. Toutefois, et ainsi que dit plus haut, ces valeurs limites ne sont que des indications, et il est possible de préparer des compositions pour administrer plus ou moins de produit.

Le variant présent dans la composition selon l'invention se lie de façon spécifique à une cible d'intérêt. De fait, il peut avoir été sélectionné, par une méthode telle que décrite dans WO 2008/068637 ou WO 2007/139397 pour sa spécificité de liaison avec cette cible d'intérêt. Par ailleurs, les méthodes décrites dans ces demandes de brevets permettent d'obtenir des affinités de l'ordre du micromolaire (WO 2007/139397) ou du nanomolaire (WO 2008/068637).

Les cibles d'intérêt sont choisies en fonction de la maladie que l'on souhaite traiter. On peut ainsi citer tout antigène, anticorps, protéine cellulaire, protéine circulante, peptides. On peut également cibler un principe actif de médicament, ou un acide nucléique particulier. On envisage en particulier que la cible d'intérêt soit une interleukine, une cytokine, un récepteur aux cytokines ou aux interleukines, une protéine codée par un oncogène, une protéine de surface d'un microorganisme, ou un lipopolysaccharide de microorganisme.

La composition pour utilisation thérapeutique ou diagnostique selon l'invention peut se présenter sous toute forme connue dans l'art. En particulier, elle se présente sous la forme de gélules, de comprimés (pelliculés ou non), de pilules ou de tablettes. Dans un autre mode de réalisation, elle se présente sous la forme d'une composition liquide, telle qu'un sirop. Elle peut également se présenter sous la forme solide,

Dans le cas où la composition se présente sous une forme solide, on peut utiliser tout excipient connu dans l'art, tel que le talc (E553b), la cellulose microcristalline, le lactose, l'amidon (en particulier de maïs), le stéarate de magnésium (E572), l'acide stéarique (E570).la cellulose microcristalline. Lorsque la composition se présente sous la forme d'un comprimé pelliculé, ladite pellicule peut être formée de toute substance connue dans l'art, telle que l'hypromellose (E464), l'éthylcellulose, la macrogol, le Talc (E553b), le dioxyde de titane (E171), l'oxyde de fer (E172).

Les compositions peuvent aussi être à libération retardée (*slow release*). De telles formulations à libération contrôlée ou retardée sont décrites, en particulier, dans WO 2011/077239. Ainsi, ces compositions peuvent inclure des matrices dans lesquelles sont présents les protéines à pli OB (naturelles ou les variants), éventuellement avec d'autres ingrédients. Ces matrices peuvent être de la sorte qu'elles permettent une libération immédiate d'une partie des protéines à pli OB, suivie d'une libération retardée d'une autre partie de ces protéines.

Dans les compositions orales se présentant sous forme solide, les protéines peuvent être lyophilisées ou intégrées dans des matrices telles que décrites dans WO 98/043615.

Il convient toutefois de noter que les exemples montrent bien que les variants des protéines à pli-OB et en particulier lorsque la protéine à pli OB est de la famille de Sac7d (SEQ ID N° 1 à SEQ ID N° 7) sont résistantes à la dégradation gastriques et peuvent donc opérer leurs effets au niveau de l'intestin, voire de la circulation systémique. Il est donc clair que, même si les éléments de la composition pour administration par voie orale peuvent permettre d'améliorer la protection de ces protéines, cela est juste un plus, mais pas un élément essentiel à la réalisation de l'effet technique.

Lorsque les compositions sont sous forme liquide, les protéines sont généralement présentes directement dans la solution.

On utilise généralement du PBS (tampon phosphate salin, soluté physiologique contenant du chlorure de sodium, du phosphate disodique, du phosphate monopotassique et un peu de chlorure de potassium.), du TBS (de l'anglais *Tris-buffered saline*), du tampon citrate (fabriqué notamment avec du citrate monosodique, 100 mM), ou de l'HEPES (acide 4-(2-hydroxyéthyl)-1-pipérazine éthane sulfonique, composé organique zwitterionique, tampon présentant une bonne stabilité au pH physiologique).

Hormis le tampon citrate pour lequel le pH est ajusté entre 4 et 6, les tampons sont préférentiellement à ph 7,4.

Toutefois, il est possible d'utiliser d'autres types de tampons pour préparer les compositions liquides orales.

Bien entendu, il est préférable que la préparation de ces tampons soit effectuée dans des conditions permettant une administration à des patients (traçabilité, bonnes pratiques de fabrication...).

Les variants des protéines à pli-OB utilisés dans la composition selon l'invention peuvent être produits par synthèse chimique en phase solide ou par recombinaison génétique. La synthèse chimique peut être réalisée par exemple avec un synthétiseur automatique de peptide du type Applied Biosystems, mod. 433A., ou en chimie Fmoc qui utilise le groupement fluoremylméthyloxycarbonyle pour la protection temporaire de la fonction α-aminique des acides aminés.

Il est toutefois préféré de produire les variant utilisables dans le cadre de l'invention par génie génétique, en particulier en intégrant une séquence d'acide nucléique codant pour ledit polypeptide dans un vecteur d'expression. Ce vecteur d'expression est alors introduit dans une cellule hôte (les bactéries telles *Escherichia coli* sont particulièrement adaptées), qui est cultivée dans des conditions de culture permettant la synthèse du polypeptide (on peut notamment utiliser des promoteurs inductibles en amont du polypeptide dans le vecteur d'expression). Le polypeptide synthétisé est alors récupéré. On peut alors greffer tout type de molécules en N- ou C-terminal du polypeptide.

L'homme du métier connaît les méthodes de production de polypeptides, qui sont notamment décrites dans l'ouvrage de Sambrook, Fritsch et Maniatis, MOLECULAR CLONING, A LABORATORY MANUAL, 2nde édition.

La composition selon l'invention peut contenir des variants de la protéine à pli-OB seuls ou avec d'autres principes actifs. Ainsi, la composition peut aussi contenir au moins un agent choisi parmi les agents antibactériens, antiparasitaires, antifongiques, anti-inflammatoires, antiprurigineux, anesthésiques, antiviraux, kératolytiques, anti-radicaux libres.

Cette composition peut également contenir un agent régulateur de pH, notamment pour permettre la régulation du pH autour de pH 7.

Les variants de la protéine à pli-OB peuvent aussi être fusionnés avec une protéine active ou tout autre composé, notamment afin d'augmenter leur temps de demi-vie.

La composition selon l'invention peut aussi être utilisée dans une méthode de diagnostic. Ainsi, dans un mode de réalisation particulier, le variant de la protéine à pli-OB est couplé à un agent de détection ou de contraste, un traceur ou un métal. Ceci permet notamment un suivi de cet agent de détection (agent de contraste, traceur, métal) et de déterminer les organes sur lesquels le variant de la protéine à pli-OB se lie (ce qui indique la présence de la cible d'intérêt). Cette composition permet alors une méthode de détection de la présence d'un antigène spécifique dans un organe, notamment dans l'appareil gastro-intestinal, comprenant l'étape d'administration d'une composition par voie orale telle que décrite ci-dessus, ladite composition comprenant un variant d'une protéine à pli-OB se liant audit antigène, et couplé à un traceur détectable (en particulier la détection des émissions de rayonnements émis par le traceur, ou un métal, ou un agent de contraste). Cette méthode peut aussi comprendre l'étape de détection de la présence du traceur dans le corps humain, et de localisation de l'organe dans lequel ce traceur est détecté, permettant de conclure à la présence de l'antigène dans l'organe où ce traceur est détecté. Cette conclusion quant à la présence de l'antigène dans un organe est clé pour conclure cette méthode de diagnostic, car elle permet de conclure quant à l'existence d'une pathologie particulière, en particulier si l'antigène est un marqueur de cette pathologie. Les traceurs les plus communs sont des isotopes (de demi-vie de moins de deux heures), tel le fluor 18 (¹⁸F) ou d'autres radioéléments tels que les ¹⁵O, ¹³N et ¹¹C.

L'invention se rapporte aussi à un procédé de préparation d'une composition pour administration par voie orale, comprenant l'étape de mélanger un variant d'une protéine sauvage à pli-OB à un excipient pour administration par voie orale, pharmaceutiquement acceptable.

L'invention se rapporte également à une composition pour administration par voie orale, selon l'invention, contenant un variant d'une protéine sauvage à pli-OB, ledit variant présentant entre 5 et 32 résidus mutés dans l'interface de liaison de ladite protéine sauvage à pli-OB à son ligand naturel, en tant que médicament. Dans ce cas particulier ledit variant se liant de façon spécifique à une cible d'intérêt, qui est une cible thérapeutique.

L'invention se rapporte également à une composition pour administration par voie orale selon l'invention pour le traitement d'une maladie ou d'un désordre systémique ou plus spécifiquement d'une maladie ou d'un désordre du système gastro-intestinal. Dans ce mode de réalisation, le variant de la protéine à pli-OB utilisé cible (se lie à) un facteur étiologique de la maladie visée ou une cible thérapeutique liée à cette maladie.

Une maladie du système gastro-intestinal est une maladie notamment choisie parmi les amibiase, cancers du côlon et du rectum, cancers de l'estomac et du duodénum, cancers de l'œsophage, maladies inflammatoires de l'appareil digestif (MICI), cancer du pancréas, choléra, hépatites et cirrhoses du foie (notamment hépatiques dues aux virus B ou C), colites (notamment dues à *Closrtidium*), diarrhées (notamment bactériennes (en particulier *Shigella, Campylobacter, Salmonella, Yersinia pseudotuberculosis* et *Yersinia enterocolitica*) ou virales (en particuliers dues aux parvovirus et réovirus, rotavirus (Réo-like virus), entérovirus, astrovirus, corona-like virus), maladie cœliaque de l'adulte, maladie de Whipple, pancréatite, syndrome du côlon irritable, tumeurs abdominales, tumeurs du foie, ulcère du duodénum ou de l'estomac, vers intestinaux, syndrome de Zollinger et Ellison.

Une maladie systémique est une maladie localisée dans un organe autre que le système gastro-instestinal.

Dans ce mode de réalisation, on sélectionne donc un variant de la protéine à pli-OB, notamment par les méthodes de criblage décrites dans WO 2008/068637, qui se lie à une cible thérapeutique (effecteur ou facteur étiologique) impliquée dans la maladie ou le désordre que l'on souhaite traiter, et on formule ce variant dans une composition pour administration par voie orale.

L'invention se rapporte également à une méthode de traitement d'une maladie ou d'un désordre systémique ou plus spécifiquement d'une maladie ou d'un désordre du système gastro-intestinal, caractérisée en ce que l'on administre par voie orale une composition selon l'invention, contenant une quantité thérapeutique efficace d'une protéine à pli-OB ciblant (se liant à), de préférence, un facteur étiologique ou une cible thérapeutique caractéristique de ladite maladie ou dudit désordre.

### Exemples

Dans l'ensemble des exemples, on utilise des nanofitines, telles que définies ci-dessus, c'est-à-dire des variants de Sac7d, obtenus par criblage d'une banque combinatoire contre une cible d'intérêt, selon une méthode similaire à la méthode décrite dans WO 2008/068637. L'objectif de ces exemples étant de démontrer que les protéines à pli-OB, et notamment les nanofitines sont capables de résister à la dégradation dans l'estomac et avoir un effet directement sur le site du système gastro-intestinal (l'intestin dans l'exemple) où leur action est nécessaire.

### Exemple 1 : test d'efficacité de nanofitines administrées par voir orale

On a testé trois nanofitines A1, A2, A3 issues de criblages d'une banque de variants de Sac7d pour leur affinité contre une cible d'intérêt, impliquée dans une maladie du système gastro-intestinal (inflammation de l'intestin). Ces nanofitines ont été produites dans un système bactérien, soit sous la forme d'une fusion avec une étiquette polyhistine, soit en l'absence de fusion (non-étiquetée).

### On a suspendu les nanofitines dans du PBS (tampon phosphate salin)

On a testé ces nanofitines sur un modèle murin d'inflammation intestinale (colite) induite au TNBS (acide 2,4,6-Trinitrobenzenesulfonique), tel que décrit dans Scheiffele et Fuss (Curr Protoc Immunol. 2002 Aug;Chapter 15:Unit 15.19), soit en mode préventif avec l'application d'une dose de nanofitine par jour à compter de 5 jours avant induction au TNBS et un sacrifice des souris 2 jours après, soit en mode curatif avec application d'une dose de nanofitine par jour à compter du jour d'induction au TNBS et un sacrifice des souris 4 jour après.

L'effet des nanofitines a été évalué au niveau macroscopique et au niveau histologique. Pour l'évaluation au niveau macroscopique, le colon a été examiné sous un microscope de dissection (x5) afin d'évaluer les lésions selon les critères de Wallace. Le score de Wallace évalue les lésions macroscopiques sur une échelle de 0 à 10 sur la base de critères reflétant l'inflammation (0 correspondant à l'absence d'inflammation et 10 à une inflammation aggravée), tels que l'hyperémie, le nombre et l'étendue des ulcérations (Pierre Desreumaux, 2001, J Exp Med. ,193, 827 - 838). Pour l'évaluation au niveau histologique, un morceau de colon situé à 2 cm au-dessus du canal anal a été découpé, puis fixé sur la nuit dans du paraformaldéhyde à 4% et intégré dans du paraffine. Les sections marquées à l'hématoxyline et à l'éosine ont été examinées « en aveugle » et évaluées selon les critères d'Ameho. Le score d'Ameho évalue les lésions sur une échelle de 0 à 6 sur la base de critère reflétant l'inflammation (0 correspondant à l'absence d'inflammation et 6 à une inflammation aggravée) tels que l'infiltration cellulaire, la profondeur et extension à la surface de la lésion (Pierre Desreumaux,2001, J Exp Med. ,193, 827 - 838). Dans les deux cas, les résultats ont été convertis en pourcentage d'efficacité reflétant le pourcentage de diminution du score par rapport au score global des souris induites non traitées.

On a utilisé, en tant que contrôle, une molécule de référence commercialisé comme anti-inflammatoire intestinal, le Pentasa® (5-amino-salicylique), utilisé à sa dose optimale. Les résultats sont indiqués à titre de référence plus que de comparaison, les dosages et voie d'administration n'étant pas rigoureusement identiques à ceux appliqués pour les nanofitines.

Appliqué de façon préventive par voie rectale (30 mM), le Pentasa® donne une efficacité de 37 % (niveau macroscopique) et 29 % (niveau histologique).

Appliqué dans le modèle curatif (présence *ad libitdum* dans la nourriture des animaux), le Pentasa® donne une efficacité de 59 % (niveau macroscopique) et 39 % (niveau histologique).

**Tableau I - Résultats obtenus sur modèle d'inflammation intestinale (pourcentage d'efficacité)**

| | | | **A1** | | **A2** | | **A3** | | **A3 nonétiquetée** | |
|---|---|---|---|---|---|---|---|---|---|---|
| | Préventif / Curatif | Dose (mg/kg) | **M** | **H** | **M** | **H** | **M** | **H** | **M** | **H** |
| | Préventif | 10 | | | | | **27,8** | **33,6** | | |
| A3 | | 100 | | | | | **18,2** | **28,1** | | |
| | | 400 | | | | | **48,6** | **45,1** | | |
| | | | | | | | | | | |
| A3 non-étiquetée | Préventif | 10 | | | | | | | **32** | **26** |
| | | 100 | | | | | | | **65** | **75** |
| | | 400 | | | | | **54** | **61** | **56** | **70** |
| | | | | | | | | | | |
| Efficacité dans modèle curatif | Curatif | 100 | **32** | **43** | **26** | **30** | **37** | **43** | | |

| | | | | | | | | | | |
|---|---|---|---|---|---|---|---|---|---|---|
| M : efficacité au niveau macroscopique H : efficacité au niveau histologique | | | | | | | | | | |

Ces résultats montrent que les nanofitines sont capables d'agir directement sur la cible localisée dans l'intestin après administration par voie orale, qu'elles traversent effectivement l'estomac sans être dégradées, et qu'elles présentent des niveaux d'efficacité comparable avec le produit de référence.

## Revendications

1. Composition pour administration par voie orale pour un usage thérapeutique ou diagnostic, comprenant un variant d'une protéine sauvage à pli-OB, ledit variant présentant entre 5 et 32 résidus mutés dans l'interface de liaison de ladite protéine sauvage à pli-OB à son ligand naturel.

2. Composition pour utilisation selon la revendication 1, **caractérisée en ce que** ladite protéine sauvage à pli-OB est choisie parmi Sac7d or Sac7e issues de *Sulfolobus acidocaldarius,* Sso7d issue de *Sulfolobus solfataricus,* DBP 7 issue de *Sulfolobus tokodaii,* Ssh7b issue de *Sulfolobus shibatae,* Ssh7a issue de *Sulfolobus shibatae,* et p7ss issue de *Sulfolobus solfataricus.*

3. Composition pour utilisation selon l'une des revendications 1 à 2, **caractérisée en ce que** ledit variant se lie à une cible d'intérêt choisie parmi les antigènes, les anticorps, les protéines cellulaires, les protéines circulantes, les peptides, les principes actifs de médicaments, les acides nucléiques, et notamment les interleukines, les cytokines, les récepteurs aux cytokines ou interleukines, les protéines codées par des oncogènes, les protéines de surfaces de microorganismes, et les lipopolysaccharides de microorganismes.

4. Composition pour utilisation selon l'une des revendications 1 à 3, **caractérisée en ce qu'**elle se présente sous la forme de gélules, de comprimés, de tablettes ou de pilules.

5. Composition pour utilisation selon l'une des revendications 1 à 3, **caractérisé en ce qu'**elle se présente sous la forme d'une composition liquide.

6. Composition pour utilisation selon l'une des revendications 1 à 5, **caractérisée en ce qu'**elle contient en outre au moins un agent choisi parmi les agents antibactériens, antiparasitaires, antifongiques, anti-inflammatoires, antiprurigineux, anesthésiques, antiviraux, kératolytiques, anti-radicaux libres.

7. Composition selon l'une des revendications 1 à 6, pour son utilisation dans le traitement d'un désordre ou d'une maladie du système gastro-intestinal.

8. Procédé de préparation d'une composition pour administration par voie orale selon l'une des revendications 1 à 7, comprenant l'étape de mélanger un variant d'une protéine sauvage à pli-OB, ledit variant présentant entre 5 et 32 résidus mutés dans l'interface de liaison de ladite protéine sauvage à pli-OB à son ligand naturel à un véhicule pharmaceutiquement acceptable pour une administration par voie orale.

9. Composition pour utilisation selon l'une des revendications 1 à 5, ladite composition comprenant un variant d'une protéine à pli-OB se liant à un antigène dont on souhaite détecter la présence dans un organe, notamment dans l'appareil gastro-intestinal, le variant étant couplé à un traceur.

## Patentansprüche

1. Zusammensetzung zur oralen Verabreichung für therapeutische oder diagnostische Zwecke, umfassend eine Variante eines Wildtyp-OB-Faltproteins, wobei die Variante zwischen 5 und 32 mutierte Reste in der Schnittstelle der Bindung des Wildtyp-OB-Faltproteins an seinen natürlichen Liganden aufweist.

2. Zusammensetzung zur Verwendung nach Anspruch 1, **dadurch gekennzeichnet, dass** das Wildtyp-OB-Faltprotein ausgewählt ist aus Sac7d oder Sac7e, abgeleitet von Sulfolobus acidocaldarius, Sso7d, abgeleitet von Sulfolobus solfataricus, DBP 7, abgeleitet von Sulfolobus tokodaii, Ssh7b, abgeleitet von Sulfolobus shibatae, Ssh7a, abgeleitet von Sulfolobus shibatae, und p7ss, abgeleitet von Sulfolobus solfataricus.

3. Zusammensetzung zur Verwendung nach einem der Ansprüche 1 und 2, **dadurch gekennzeichnet, dass** die Variante an ein Target von Interesse bindet, das ausgewählt ist aus Antigenen, Antikörpern, Zellproteinen, zirkulierenden Proteinen, Peptiden, Wirkstoffen von Arzneimitteln, Nukleinsäuren, insbesondere Interleukinen, Zytokinen, Zytokin- oder Interleukinrezeptoren, durch Onkogene kodierten Proteinen, Oberflächenproteinen von Mikroorganismen und Lipopolysacchariden von Mikroorganismen.

4. Zusammensetzung zur Verwendung nach einem der Ansprüche 1 bis 3, **dadurch gekennzeichnet, dass** sie in Form von Gelkapseln, Tabletten, Rauten oder Pillen vorliegt.

5. Zusammensetzung zur Verwendung nach einem der Ansprüche 1 bis 3, **dadurch gekennzeichnet, dass** sie in Form einer flüssigen Zusammensetzung vorliegt.

6. Zusammensetzung zur Verwendung nach einem der Ansprüche 1 bis 5, **dadurch gekennzeichnet, dass** sie auch mindestens ein Mittel enthält, das ausgewählt ist aus antibakteriellen, antiparasitären, antimykotischen, entzündungshemmenden, antipruritischen, betäubenden, antiviralen, keratolytischen und radikalischen Mitteln.

7. Zusammensetzung nach einem der Ansprüche 1 bis 6 zur Verwendung bei der Behandlung einer Störung oder Erkrankung des Magen-Darm-Systems.

8. Verfahren zur Herstellung einer oralen Verabreichungszusammensetzung nach einem der Ansprüche 1 bis 7, umfassend den Schritt des Mischens einer Variante eines Wildtyp-OB-Faltproteins, wobei die Variante zwischen 5 und 32 mutierte Reste in der Schnittstelle der Bindung des Wildtyp-OB-Faltproteins an seinen natürlichen Liganden mit einem pharmazeutisch annehmbaren oralen Verabreichungsträger aufweist.

9. Zusammensetzung zur Verwendung nach einem der Ansprüche 1 bis 5, wobei die Zusammensetzung eine Variante eines OB-Faltproteins umfasst, das an ein Antigen bindet, dessen Anwesenheit man in einem Organ, insbesondere im Magen-Darm-System, nachweisen möchte, wobei die Variante an einen Tracer gekoppelt ist.

## Claims

1. An composition for oral administration for therapeutic or diagnostic use comprising a variant of a wild-type OB-fold protein, said variant having between 5 and 32 mutated residues in the interface of binding of said wild-type OB-fold protein to its natural ligand.

2. The composition for use as claimed in claim 1, **characterized in that** said wild-type OB-fold protein is chosen from Sac7d or Sac7e derived from *Sulfolobus acidocaldarius,* Sso7d derived from *Sulfolobus solfataricus,* DBP 7 derived from *Sulfolobus tokodaii,* Ssh7b derived from *Sulfolobus shibatae,* Ssh7a derived from *Sulfolobus shibatae,* and p7ss derived from *Sulfolobus solfataricus.*

3. The composition for use as claimed in either of claims 1 and 2, **characterized in that** said variant binds to a target of interest chosen from antigens, antibodies, cell proteins, circulating proteins, peptides, active ingredients of medicaments, nucleic acids, and in particular interleukins, cytokines, cytokine or interleukin receptors, proteins encoded by oncogenes, surface proteins of microorganisms, and microorganism lipopolysaccharides.

4. The composition for use as claimed in one of claims 1 to 3, **characterized in that** it is in the form of gel capsules, tablets, lozenges or pills.

5. The composition for use as claimed in one of claims 1 to 3, **characterized in that** it is in the form of a liquid composition.

6. The composition for use as claimed in one of claims 1 to 5, **characterized in that** it also contains at least one agent chosen from antibacterial, antiparasitic, antifungal, antiinflammatory, antipruritic, anesthetic, antiviral, keratolytic and free-radical-scavenger agents.

7. The composition as claimed in one of claims 1 to 6, for use thereof in treating a gastrointestinal system disorder or disease.

8. A process for preparing an oral administration composition as claimed in one of claims 1 to 7, comprising the step of mixing a variant of a wild-type OB-fold protein, said variant having between 5 and 32 mutated residues in the interface of binding of said wild-type OB-fold protein to its natural ligand with a pharmaceutically acceptable oral administration carrier.

9. A composition for use according to any one of claims 1 to 5, said composition comprising a variant of an OB-fold protein which binds to an antigen the presence of which one wishes to detect in an organ, in particular in the gastrointestinal system, wherein said variant is coupled to a tracer.


### SEQUENCE LISTING

<110> AFFILOGIC
<120> COMPOSITIONS POUR ADMINISTRATION PAR VOIE ORALE COMPRENANT UN VARIANT D'UNE PROTÉINE À PLI-OB
<130> BRV 95 - WO
<150> FR 14/60226
   <151> 2014-10-24
<160> 8
<170> PatentIn version 3.3
<210> 1
   <211> 66
   <212> PRT
   <213> Sulfolobus acidocaldarius
<400> 1
   Met Val Lys Val Lys Phe Lys Tyr Lys Gly Glu Glu Lys Glu Val Asp
   1               5                   10                  15
   Thr Ser Lys Ile Lys Lys Val Trp Arg Val Gly Lys Met Val Ser Phe
               20                  25                  30
   Thr Tyr Asp Asp Asn Gly Lys Thr Gly Arg Gly Ala Val Ser Glu Lys
           35                  40                  45
   Asp Ala Pro Lys Glu Leu Leu Asp Met Leu Ala Arg Ala Glu Arg Glu
       50                  55                  60
   Lys Lys
   65
<210> 2
   <211> 64
   <212> PRT
   <213> Sulfolobus solfataricus
<400> 2
   Met Ala Thr Val Lys Phe Lys Tyr Lys Gly Glu Glu Lys Glu Val Asp
   1               5                   10                  15
   Ile Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
                     50                  55                  60
<210> 3
   <211> 65
   <212> PRT
   <213> Sulfolobus acidocaldarius
<400> 3
   Met Ala Lys Val Arg Phe Lys Tyr Lys Gly Glu Glu Lys Glu Val Asp
   1               5                   10                  15
   Thr Ser Lys Ile Lys Lys Val Trp Arg Val Gly Lys Met Val Ser Phe
               20                  25                  30
   Thr Tyr Asp Asp Asn Gly Lys Thr Gly Arg Gly Ala Val Ser Glu Lys
           35                  40                  45
   Asp Ala Pro Lys Glu Leu Met Asp Met Leu Ala Arg Ala Glu Lys Lys
       50                  55                  60
   Lys
   65
<210> 4
   <211> 64
   <212> PRT
   <213> Sulfolobus shibatae
<400> 4
   Met Val Thr Val Lys Phe Lys Tyr Lys Gly Glu Glu Lys Glu Val Asp
   1               5                   10                  15
   Thr Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 5
   <211> 64
   <212> PRT
   <213> Sulfolobus shibatae
<400> 5
   Met Ala Thr Val Lys Phe Lys Tyr Lys Gly Glu Glu Lys Gln Val Asp
   1               5                   10                  15
   Ile Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 6
   <211> 64
   <212> PRT
   <213> Sulfolobus sp.
<400> 6
   Met Val Thr Val Lys Phe Lys Tyr Lys Gly Glu Glu Lys Glu Val Asp
   1               5                   10                  15
   Ile Ser Lys Ile Lys Lys Val Trp Arg Val Gly Lys Met Ile Ser Phe
               20                  25                  30
   Thr Tyr Asp Asp Asn Gly Lys Thr Gly Arg Gly Ala Val Ser Glu Lys
           35                  40                  45
   Asp Ala Pro Lys Glu Leu Leu Gln Met Leu Glu Lys Ser Gly Lys Lys
       50                  55                  60
<210> 7
   <211> 64
   <212> PRT
   <213> Sulfolobus islandicus
<400> 7
   Met Val Thr Val Lys Phe Lys Tyr Lys Gly Glu Glu Lys Gln Val Asp
   1               5                   10                  15
   Thr Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 8
   <211> 60
   <212> PRT
   <213> Artificial
<220>
   <223> Séquence consensus
<220>
   <221> VARIANT
   <222> (2)..(2)
   <223> Xaa est V, A ou T
<220>
   <221> VARIANT
   <222> (3)..(3)
   <223> Xaa est T ou K
<220>
   <221> VARIANT
   <222> (5)..(5)
   <223> Xaa est R ou K
<220>
   <221> VARIANT
   <222> (14)..(14)
   <223> Xaa est E ou Q
<220>
   <221> VARIANT
   <222> (17)..(17)
   <223> Xaa est T ou I
<220>
   <221> VARIANT
   <222> (30)..(30)
   <223> Xaa est A, V ou I
<220>
   <221> VARIANT
   <222> (36)..(38)
   <223> Xaa Xaa Xaa est EGG ou DN-
<220>
   <221> VARIANT
   <222> (56)..(56)
   <223> Xaa est M ou L
<220>
   <221> VARIANT
   <222> (57)..(57)
   <223> Xaa est Q ou D
<220>
   <221> VARIANT
   <222> (60)..(60)
   <223> Xaa représente A, ARAE, EKQKK, EKSGKK, ARAEREKK, ARAEKKK, ACAEREKK ou ACAEKKK
<400> 8
   Met Xaa Xaa Val Xaa Phe Lys Tyr Lys Gly Glu Glu Lys Xaa Val Asp
   1               5                   10                  15
   Xaa Ser Lys Ile Lys Lys Val Trp Arg Val Gly Lys Met Xaa Ser Phe
               20                  25                  30
   Thr Tyr Asp Asp Asn Xaa Gly Lys Thr Gly Arg Gly Ala Val Ser Glu
           35                  40                  45
   Lys Asp Ala Pro Lys Glu Leu Xaa Xaa Met Leu Xaa
       50                  55                  60